**Europäisches Patentamt**

**European Patent Office**

**Office européen des brevets**

(19)

(11) Publication number: **0 125 893**

**A2**

(12) # EUROPEAN PATENT APPLICATION

(21) Application number: **84303171.7**

(22) Date of filing: **10.05.84**

(51) Int. Cl.³: **G 01 N 33/54**
**G 01 N 33/74**

(30) Priority: **12.05.83 JP 83730/83**
**07.03.84 JP 43599/84**
**08.03.84 JP 44942/84**

(43) Date of publication of application:
**21.11.84 Bulletin 84/47**

(84) Designated Contracting States:
**DE FR GB SE**

(71) Applicant: SUMITOMO CHEMICAL COMPANY, LIMITED
15 Kitahama 5-chome Higashi-ku
Osaka-shi Osaka-fu(JP)

(72) Inventor: Noguchi, Hiroshi
4-153 Seiwadai-Nishi 4-chome
Nishinomiya-shi Hyogo-ken(JP)

(72) Inventor: Nakanishi, Toru
8-3-205 Hamakoshien 3-chome
Nishinomiya-shi Hyogo-ken(JP)

(72) Inventor: Ogino, Shigeo
30-202 Koshikiiwa-cho 5-chome
Nishinomiya-shi Hyogo-ken(JP)

(74) Representative: Allard, Susan Joyce et al,
BOULT, WADE & TENNANT 27 Furnival street
London EC4A 1PQ(GB)

(54) The quantitative analysis of antigen by the enzyme-antibody bridge method.

(57) An improved method for the quantitative analysis of antigen by an enzyme-antibody bridge method, which comprises binding (1) an insoluble carrier or a coated insoluble carrier with (2) an antibody being capable of recognizing an antigen (first antibody), (3) an antigen to be determined, (4) an antibody being capable of recognizing an antigen originated from an animal species different from the animal species of the first antibody (second antibody), (5) an anti-immunoglobulin antibody being capable of recognizing an immunoglobulin of the same animal species as that of the second antibody, (6) an anti-enzyme antibody prepared by using the same animal species as that of the second antibody, (7) an enzyme in this order, and then measuring the amount of the enzyme in the resulting enzyme-anti-enzyme antibody complex, and determining the amount of the antigen therefrom. Said improved method gives a high sensitivity and hence can also be used for the measurement of very low concentration of an antigen.

Croydon Printing Company Ltd

# THE QUANTITATIVE ANALYSIS OF ANTIGEN BY

# THE ENZYME-ANTIBODY BRIDGE METHOD

The present invention relates to an enzyme immunoassay for the quantitative analysis of an antigen contained in a test sample.

The quantitative analysis of antigens has widely been used for purposes such as the diagnosis of various diseases and the quality control of products in purification processes in industry. For example, by the quantitative analysis of hormones in body samples such as blood, the endocrinic functions of the body can be diagnosed. The quantitative analysis of virus antigens or tumor-associated antigens is useful for the diagnosis of infectious diseases or cancer and also for the observation of progress of the diseases. Moreover, the measurement of antigens is also useful for the determination of the amount of biologically active components in microorganisms and animal cells and further for the quality control of the biologically active components in certain purification processes.

Various methods for the quantitative analysis of antigens are known, such as a method of visually measuring the extent of precipitation or agglutination of antigen-antibody reaction, a radioimmunoassay (RIA) and an enzyme immunoassay (EIA). However, the method of visual determination of the extent of precipitation or agglutination requies a high degree of skill in its operation and takes a much longer time for the determination and has less accuracy. The RIA and EIA methods are easy to operate with high accuracy and good sensitivity and hence are widely used in various fields. In particular, EIA is advantageous it its safety over RIA since no hazardous radio-isotope is used. However, in the conventional EIA method, the enzyme is directly bound to the antibody via a covalent bond and the antibody is thereby denatured, which results in a decrease in the sensitivity of the EIA method.

We have now developed an improvement of specificity and sensitivity in the EIA method so that even a low concentration of antigen can be specifically determined.

Accordingly, the present invention provides a method for the quantitative determination of an antigen which comprises binding (1) an insoluble carrier or a coated insoluble carrier with (2) an antibody which is capable of recognizing an antigen (first antibody), (3) an antigen to be determined, (4) an antibody (second antibody) which is capable of recognizing an antigen and originating from an animal species different from the animal species, from which the first antibody is obtained, (5) an anti-immunoglobulin antibody which is capable of recoginzing an immunoglobulin of the same animal species as that from which the second antibody is obtained, (6) an anti-enzyme antibody prepared by using the same animal species as that from which the second antibody is obtained, and (7) an enzyme in this order, measuring the amount of the enzyme in the resulting enzyme-anti-enzyme antibody complex and determining the amount of the antigen therefrom.

The insoluble carrier used in the present invention may be a shaped plastic such as polyvinyl chloride, polystyrene or polyethylene, which may be for example, a plate, microplate, vessel (e.g. test tube) or beads . Of these, microplates made of polyvinyl chloride are particularly preferred because they have a large capacity of binding to the first antibody and are easily washed. By washing the insoluble carrier or by coating it with a coating agent selected from hemagglutinins such as concanavalin A (Con A) and phytohemagglutinin (PHA), polysaccharides such as dextran, amino acid mono and heteropolymer, or microbial cells containing, for example, protein A, the amount of the antibody bound thereto can be increased, which, in turn, results in an increase of the binding of the antigen in the test sample and hence in an increase in the sensitivity of the determination.

The coating of the insoluble carrier with a coating agent can be carried out as follows. A solution of a coating agent (for example, PHA-P, No. L-1800, manufactured by E.Y. Laboratories) (10 to 500 $\mu$g/ml) in water or a buffer solution (e.g. phosphate buffered saline, abbreviated as "PBS") is charged into a vessel-shaped insoluble carrier or a vessel containing the insoluble carrier, and the mixture is incubated at 4 to 37°C for 15 minutes to 2 hours, whereby the insoluble carrier can be coated by the coating agent. For example, in the case of a commercially available microplate with 96 wells (e.g. FALCON # 3912 Microtest III flexible assay plate, manufactured by FALCON), about 50 to

200 $\mu$l of the coating agent is charged to each well. After removing the coating agent-containing solution, the carrier is dried at 20 to 37°C, and then washed with a buffer solution (e.g. PBS) 1 to 4 times.

The antibody which is capable of recognizing an antigen (the first antibody) may be an anti-serum (polyclonal antibody) against the antigen, a monoclonal antibody, or a mixture of monoclonal antibodies each having a different specificity of antigen binding. The first antibody may be used as an unpurified preparation or after purification by conventional purification methods which are usually used in the biological field, for example, fractionation with ammonium sulfate, ion exchange chromatography, affinity chromatography, etc. Furthermore the first antibody may be a whole molecule of immunoglobulin or may be a $F(ab')_2$ fragment thereof. The first antibody should originate from an aminal species different from the one from which the anti-enzyme antibody is obtained.

The anti-serum useful as the first antibody can be prepared by immunizing an animal (e.g. rabbit, goat or sheep) with the antigen by a conventional method. The monoclonal antibody can be prepared by immunizing an animal (e.g. mouse or rat) with an antigen, fusing the antibody-producing cells such as spleen cells of the immunized animal with myeloma cells of mice or rats to obtain fused cells (hybridoma), cloning the hybridoma, culturing the hybrid cell clones in vitro or growing the clones in abdominal ascites of an animal [cf. Köhler et al.,

Nature, 256, 495 (1975), Köhler et al., Eur. J. Immunol., 6, 511 (1976), Köhler et al., Somatic Cell Genetics, 3, 303 (1977), and Japanese Patent Application No. 160516/1982].

The first antibody can be bound to the insoluble carrier or a coated insoluble carrier in the following manner. For example, a solution of the first antibody (10 to 500 $\mu$g/ml) in a buffer solution (e.g. PBS) is added to an insoluble carrier for the commercially available microplate, 50 to 200 $\mu$l per well), and the mixture is incubated at 20 to 37°C for 1 to 5 hours. After removing the solution, it is dried at 20 to 37°C, and a 1 to 5 % solution of bovine serum albumin (BSA) (50 to 200 $\mu$l) is added to it, and reacted at 20 to 37°C for 1to 5 hours, after which the region of the insoluble carrier unbound with the first antibody is blocked with BSA. After removing the excess amount of the BSA solution, the vessel is dried at 20 to 37°C.

The nonspecific binding of the first antibody to the insoluble carrier is effective for specifically binding a large amount of the antigen contained in the test sample to the insoluble carrier via the first antibody, and results in an increase of the sensitivity of the present method. When a first antibody having a high antibody titer, e.g. a first antibody purified by affinity column chromatography, is bound to the insoluble carrier, the sensitivity is further improved. When the antigen is directly nonspecifically bound to the insoluble carrier, the antigen is bound in less than molecular amount, and hence, the sensitivity of the determination is undesirably low.

The antigen to be measured by the present invention may be various hormones and growth factors, such as insulin, gastrin, secretin, calcitonin, adrenocorticotropic hormone, oxytocin, luteinizing hormone, growth hormone, growth hormone-releasing factor, somatostatin, thyroid hormone, somatomedines, fibroblast growth factor, epithelial cell growth factor, platelet-derived growth factor, tumor growth factor, and erythropoietine; tumor-associated antigens, such as carcinoembryonic antigen (CEA), $\alpha$-fetoprotein (AFP); specific antigens of microorganisms (e.g. bacteria, virus, fungi, protozoa), such as hepatitis B virus surface antigen (HBs antigen) or syphilis specific antigen; interferones; lymphokine, monokine, cytokine or C-reactive proteins (CRP). In the present invention, the antigens in blood plasma, serum or urine, or the antigens isolated, for example, by extraction of the samples can be measured.

The binding of the antigen to the first antibody bound to the insoluble carrier is carried out as follows: The test sample is added to the insoluble carrier bound with the first antibody prepared above (50 to 200 $\mu$l per well  for the microplate with 96 wells),  and the mixture is incubated at 20 to 37°C for 1 to 5 hours, or at 4°C overnight, or at 20 to 37°C for 1 to 5 hours and further at 4°C overnight, after which the antigen contained in the test sample is bound thereto.  After removing the test sample, the insoluble carrier bound to the first antibody and the antigen is washed with a buffer solution (e.g. PBS).

The second antibody which is capable of recognizing the antigen is an antibody against the above antigen, which is prepared by using an animal species different from that of the first antibody. The second antibody includes an anti-serum (polyclonal antibody), a monoclonal antibody, and a mixture of monoclonal antibodies. Preferably, either one or both of the first antibody and the second antibody is a monoclonal antibody. When a monoclonal antibody is used, the antibody shows a high specificity, and hence, any undesirable cross-reactivity with analogous substances can be decreased. For example, when a monoclonal anti-human growth hormone (HGH) is used, any cross-reactivity with substances analogous to HGH (e.g. prolactin, etc.) can be decreased. Furthermore, when a monoclonal anti-interferon (IFN) antibody is used, it shows a high specificity, and the cross-reactivity not only between the IFN types such as $\alpha$-, $\beta$-, and $\gamma$-IFN but also between the IFN subtypes thereof is decreased. The anti-serum and monoclonal antibody for the second antibody are prepared in the same manner as those for the first antibody. Furthermore, as the second antibody, not only the whole molecule of immunoglobulin but also the $F(ab')_2$ fragment can be used.

The binding of the second antibody to the insoluble carrier can be carried out as follows: The second antibody is diluted 50 to 5,000 times with a buffer solution containing 5 to 10 % normal animal serum and 0.1 to 0.5 % Tween 20, and the diluted second antibody solution (50 to 200 $\mu$l per well for the microplate with 96 wells) is added to the insoluble carrier bound with the antigen prepared as above. The mixture is incubated at 20 to 37°C for 1 to 5 hours and then washed with a buffer solution (e.g. PBS). Alternatively, the second antibody may be added to the insoluble carrier bound with the first antibody simultaneously with the antigen to be determined (i.e. the test sample), wherein the antigen and the second antibody are reacted with and bound to the insoluble carrier in this order. In the latter method, the test sample (e.g 50 to 200 $\mu$l per well) and a solution of second antibody (e.g. 50 to 200 $\mu$l per well) diluted 50 to 5000 times with a buffer solution containing 5 to 10 % normal animal serum and 0.1 to 0.5 % Tween 20 as above are added to the insoluble carrier bound with the first antibody, and the mixture is incubated at 20 to 37°C for 1 to 5 hours, or at 4°C overnight, or at 20 to 37°C for 1 to 5 hours and further at 4°C overnight. (TWEEN is a Registered Trade Mark).

The anti-immunoglobulin antibody is an anti-serum which can recognize immunoglobulin of the same animal species as the one from which the above second antibody is obtained. The anti-serum can be prepared by immunizing an animal of a different species from the animal species from which the above second antibody is obtained with an immunoglobulin of the same animal species as that of the second antibody by a known method. The anti-immunoglobulin antibody may be the whole molecule of immunoglobulin or the

F(ab')$_2$ fragment.

The binding of the anti-immunoglobulin antibody to the insoluble carrier can be carried out as follows: The insoluble carrier bound with the second antibody prepared as above is washed 1 to 4 times with a buffer solution (e.g. PBS). An anti-immunoglobulin antibody is diluted 50 to 5,000 times with a buffer solution containing 5 to 10 % normal animal serum and 0.1 to 0.5 % Tween 20, and added to the insoluble carrier (50 to 200 $\mu$l per well for the microplate with 96 wells), and the mixture is incubated at 20 to 37°C for 0.5 to 5 hours. Alternatively, the anti-immunoglobulin antibody may be added to the insoluble carrier simultaneously with the antigen (i.e. test sample) and the second antibody, whereby the antigen, the second antibody and the anti-immunoglobulin antibody are bound to the first antibody bound to the insoluble carrier in this order. In the latter method, the test sample (e.g. 50 to 200 $\mu$l per well), a solution of second antibody (e.g. 50 to 200 $\mu$l per well) diluted 50 to 5000 times with a buffer solution (e.g. PBS) containing 5 to 10 % normal animal serum and 0.1 to 0.5 % Tween 20 as above and an anti-immunoglobulin antibody (e.g. 50 to 200 $\mu$l per well) diluted 50 to 5000 times with the said buffer solution are added to the insoluble carrier bound with the first antibody, and the mixture is incubated at 20 to 37°C for 0.5 to 5 hours, or at 4°C overnight, or at 20 to 37°C for 0.5 to 5 hours and further at 4°C overnight.

The anti-immunoglobulin antibody may be a mixture of an anti-immunoglobulin antibody labelled with an enzyme, which is prepared by binding the enzyme to the antibody and a non-labelled anti-immunoglobulin antibody. In this case, an enzyme-labelled anti-immunoglobulin antibody (e.g. 50 to 200 $\mu$l per well) as diluted 10 to 100 times with a buffer solution (e.g. PBS) containing 5 to 10 % normal animal serum and 0.1 to 0.5 % Tween 20 and a non-labelled anti-immunoglobulin antibody (e.g. 50 to 200 $\mu$l per well) diluted 50 to 5,000 times with the said buffer soluiton are simultaneously added to the insoluble carrier, and the mixture is incubated at 20 to 37°C for 0.5 to 5 hours.

The anti-immunoglobulin antibody stands between the above second antibody and the anti-enzyme antibody mentioned hereinafter and bridges between them. The bridge is effected by an antigen-antibody reaction. The binding reaction (i.e. an enzyme-antibody bridge method) is milder in comparison with the conventional binding of enzyme to the antibody by a chemical reaction, and hence, can avoid the undesirable lowering of enzyme activity which occurs in the conventional chemical binding method. Moreover, more than one molecule of the anti-immunoglobulin antibody can bind to one molecule of the second antibody, and further more than one molecule of the anti-enzyme antibody can also bind to one molecule of the anti-immunoglobulin antibody. Thus, the number cf enzyme molecules to be bound to one molecule of the antigen contained in the test sample is amplified, and hence, the sensitivity of the present method is remarkably high. The enzyme-antibody bridge method has

- 11 -     0125893

been developed by Sternberger et al. [cf. Sternberger et al., J. of Histochemistry and Cytochemisty, 18, 315 (1970)] and has widely been used as a detection method for protein antigen in animal tissues, but this method has never been applied to the quantitative analysis of an antigen with high sensitivity and high accuracy.

The anti-enzyme antibody is an antibody which is capable of recognizing the enzyme mentioned hereinafter. The anti-enzyme antibody may be either an anti-serum (polyclonal antibody) or a monoclonal antibody, but should be prepared by using the same animal species as that of the second antibody. The anti-enzyme antibody may be the whole molecule of immunoglobulin or the $F(ab')_2$ fragment.

The anti-serum as the anti-enzyme antibody is prepared by immunizing an animal of the same species as the animal species from which the second antibody is obtained with the enzyme by a conventional method. The monoclonal antibody can be prepared by fusing anti-enzyme antibody-producing cells and myeloma cells to obtain fused cells (hybridoma) and culturing the hydridoma or by growing the hydridoma in abdominal ascites of an animal [cf. Köhler et al., Nature, 256, 495, (1975); Köhler et al., Eur. J. Immunol., 6, 511 (1976); and Köhler et al., Somatic Cell Genetics, 3, 303 (1977)].

The binding of the anti-enzyme antibody can be carried out, for example, as follows: The insoluble carrier bound with the anti-immunoglobulin antibody prepared as above is washed 1 to 4 times with a buffer solution (e.g. PBS). An anti-enzyme antibody is diluted 100 to 5,000 times with a buffer solution containing 5 to 10 % normal animal serum and 0.1 to 0.5 % Tween 20, and added to the insoluble carrier washed as above (50 to 200 $\mu$l per well in case of the microplate with 96 wells), and the mixture is incubated at 20 to 37°C for 10 minutes to 2 hours.

The enzyme used in the present invention may be any enzyme which is usually used in an enzyme immunoassay, for example, peroxidase, alkaline phosphatase or $\beta$-galactosidase. The binding of the enzyme to the insoluble carrier prepared as above can be carried out, for example, as follows: The insoluble carrier bound with the anti-enzyme antibody is washed 1 to 4 times with a buffer solution (e.g. PBS), and an enzyme solution (e.g. 50 to 200 $\mu$l per well) diluted to a concentration of 0.1 to 10 $\mu$g/ml with the said buffer solution is added to it, and the mixture is incubated at 20 to 37°C for 10 minutes to 2 hours. Alternatively, the enzyme may be added to the insoluble carrier simultaneously with the above anti-enzyme antibody, whereby the anti-enzyme antibody and the enzyme can react with and bond to the insoluble carrier in this order. In the latter method. The insoluble carrier bound with the anti-immunoglobulin antibody prepared as above is washed 1 to 4 times with a buffer solution (e.g. PBS), and an anti-enzyme antibody (e.g. 50 to 200 $\mu$l per well) diluted 100 to 5,000 times with a buffer solution containing 5 to 10 % normal animal serum and 0.1 to 0.5 % Tween 20 and an enzyme solution (e.g. 50 to 200 $\mu$l per

well) diluted in a concentration of 0.1 to 10 $\mu$g/ml with the above buffer solution are added to it, and the mixture is incubated at 20 to 37°C for 10 minutes to 2 hours.

The amount of the enzyme bound to the insoluble carrier can be determined by reacting the enzyme-anti-enzyme-antibody complex thus obtained with the corresponding substrate, and then measuring the absorbance of the reaction mixture at a wavelength corresponding to the substrate by a conventional spectrometry. Based on the amount of bound enzyme thus obtained, the amount of antigen contained in the test sample is calculated by using a calibration curve which is previously drawn from the correlation between known amounts of antigen and the absorbance of the reaction mixture corresponding to the amounts of enzyme bound to the insoluble carrier. For example, when using azino-bis(3-ethylbenzothiazolin-6-sulfonic acid) (ABTS) as the substrate, the absorbance at a wavelength of 405 nm is measured.

Thus, the present invention is characteristic in that firstly the antigen contained in the test sample is effectively bound to the insoluble carrier via the first antibody, and that secondly the second antibody and the anti-enzyme antibody are bridged with an anti-immunoglobulin antibody and the amount of enzyme bound to the anti-enzyme antibody is measured, from which the amount of the antigen contained in the test sample can be determined with high sensitivity and also quantitatively (enzyme-antibody bridge method). In the present invention, a mixture of an enzyme-labelled and non-labelled product is used as the anti-immunoglobulin antibody in order to increase the sensitivity of the measurement.

According to the present invention, an antigen contained in a test sample even in a low concentration can be measured with a high sensitivity.

When measuring hyman growth hormone (HGH), the measurable lower limit using a conventional method is 5 ng/ml or more, but that of the present invention is as low as 0.25 ng/ml.    For    interferon (IFN), the lower limit using a conventional method is 10 to 50 U ml or more, but that of the present invention is 3 U/ml. Furthermore, in the case of carcinoembryonic antigen (CEA), the lower limit of the present invention is 0.067 ng/ml which is superior to that (1 ng/ml or more) of the conventional method.  One of preferred features of the present invention is that by using a monoclonal antibody for at least one of the first antibody and the second antibody, undesirable cross-reactivity with substances analogous to the antigen to be measured is inhibited and the specificity of the binding to the antigen is enhanced.

The present invention is illustrated by the following Examples but should not be construed to be limited thereto.

Example 1

To a cleaned microplate made of polyvinyl chloride with 96 wells (FALCON # 3912 Microtest III flexible assay plate, manufactured by FALCON), a solution of a rabbit anti-human growth hormone (HGH) antiserum (100 $\mu$c ml) in a phosphate buffered saline (PBS) is added in an ar ınt of 100

μl per each well, and incubated at 37°C for 2 hours. After removing the solution, the microplate is dried at 37°C for 10 minutes, and then, a PBS (200 μl) containing 3 % bovine serum albumin (BSA) is added to each well, and reacted at 37°C for 1 hour, by which the region unreacted with anti-HGH antiserum is blocked with BSA. After removing the reaction mixture, the microplate is dried at 37°C for 10 minutes, washed three times with PBS, and 100 μl of a test sample containing HGH and 20 μl of a mouse monoclonal anti-HGH antibody (purified IgG, 120 μg/ml, commercially available from Sumitomo Chemical Co., Ltd, Japan under the name of CL·B1) in a PBS containing 5 % BSA and 0.1 % Tween 20 are added to each well, and the mixture is incubated at 4°C overnight. After removing the reaction mixture, the microplate is washed three times with PBS, and 100 μl of a rabbit anti-mouse immunoglobulin antibody diluted in 1,000 folds with a PBS containing 10 % normal rabbit serum and 0.1 % Tween 20 is added to each well and incubated at 37°C for 30 minutes. After washing three times with PBS, a mixture (100 μl) containing a 1,000 fold-diluted mouse anti-peroxidase antiserum, diluted with the above PBS and peroxidase (1 μg/ml) in the above PBS is added to each well of the microplate, and incubated at 37°C for 30 minutes. After removing the reaction mixture, the microplate is washed three times with PBS, and 100 μl of a substrate solution (12.5 mg/ml) of azino-bis(3-ethylbenzothiazoline-6-sulfonic acid) (ABTS) in a 100 mM sodium citrate-phosphate buffer (PH 5.0) solution containing 1.47 mM sodium perborate is added, and colored by reacting at 37°C for 30 minutes.

The absorbance of the colored product is measured at a wavelength of 405 nm, and the amount of HGH contained in the test sample is determined using a previously prepared calibration curve.

The calibration curve is prepared by repeating the above procedure except that a solution of a standard HGH in a PBS containing 5 % BSA and 0.1 % Tween 20 is used instead of the test sample. The following Table 1 shows the value of calibration curve and the coefficient of variation measured, and Table 2 shows the date of HGH measured in the test sample I and II and the coefficient of variation measured.

Table 1

| HGH (ng/ml) | 0.25 | 1 | 4 | 16 | 64 |
|---|---|---|---|---|---|
| Average of measured value (A 405)* | 0.036 | 0.107 | 0.388 | 0.956 | 1.078 |
| Coefficient of variation (%) | 13.9 | 4.7 | 3.9 | 3.1 | 2.8 |

*) Background value A405 = 0.071 (corrected)

Table 2

| Test sample | I (8.2 ng/ml)* | II (17 ng/ml)* |
|---|---|---|
| Average of measured value (ng/ml) | 8.8 | 17 |
| Coefficient of variation (%) | 2.9 | 1.9 |

*) The amount of HGH in the test sample was measured by using a commercially available RIA kit (HGH-1 "Eiken", manufactured by Eiken Kagaku K.K.)

As is shown in the above experimental data, the method of the present invention is effective for determination of HGH of 0.25 ng/ml or more with good reproduction, and the sensitivity for measurement is largely improved in comparison with the conventional method.

Example 2

In the same manner as described in Example 1, a test sample and a mouse monoclonal anti-HGH antibody are added to a microplate bound with a rabbit anti-HGH antiserum and BSA, and incubated at 4°C overnight. After washing three times with PBS, a mixture (100 μl) of a 100 fold-diluted rabbit anti-mouse-immunoglobulin antibody, diluted with PBS containing 10 % normal rabbit serum and 0.1 % Tween 20, and a 20 fold-diluted peroxidase-labelled sheep anti-mouse-immunoglobulin antibody, diluted with the above PBS, is added to each well, and it is incubated at 37°C for 30 minutes. After removing the reaction mixture and further washing three times with PBS, a mixture (100 μl per well) containing a 1,000 fold-diluted anti-peroxidase antiserum, diluted with PBS containing 10 % normal rabbit serum and 0.1 % Tween 20, and 1 μg/ml of peroxidase, diluted with the above PBS, is added to the microplate, and incubated at 37°C for 30 minutes. After removing the reaction mixture and washing three times with the above PBS, it is colored by adding the substrate ABTS in the same manner as described in Example 1. The calibration curve is prepared by repeating the above procedure except that a solution of a standard HGH in a PBS containing 5 % BSA and 0.1 % Tween 20 is used instead of the test sample. The following Table 3 shows the value of calibration curve and the coefficient of variation measured.

Table 3

| HGH (ng/ml) | 0.1 | 0.25 | 1 | 4 | 16 |
|---|---|---|---|---|---|
| Average of measured value (A 405)* | 0.036 | 0.146 | 0.503 | 1.178 | 1.580 |
| Coefficient of variation (%) | - | 1.0 | 1.0 | 1.8 | 3.7 |

*) Background value A405 = 0.147 (corrected)

Example 3

To a cleaned microplate made of polyvinyl chloride with 96 wells (the same kind of microplate as used in Example 1), a solution of PHA-P (No. L-1800, Lot 040478, manufactured by E Y Laboratories) (50 $\mu$g/ml) in PBS is added in an amount of 100 $\mu$l per each well, and the solution is incubated at 37°C for 1 hours. After removing the reaction mixture, the microplate thus treated is dried at 37°C for 10 minutes, and then washed three times with PBS. In the same manner as in Example 1, a rabbit anti-HGH antiserum and 3 % BSA are added in this order and bound to the microplate. After washing three times with PBS, a test sample (100 $\mu$l per well), a mouse monoclonal anti-HGH antibody (25 $\mu$l per well) diluted in 5 folds with a PBS containing 1 % BSA and 0.1 % Tween 20, and 25 $\mu$l per well of a rabbit anti-mouse-immunoglobulin antibody diluted in 100 folds with the above PBS are added to it, and incubated at 4°C overnight. After removing the reaction mixture and further washing three times with PBS, a mixture (100 $\mu$l per well) containing a

- 19 -     0125893

1,000 fold-diluted mouse anti-peroxidase antiserum, diluted with a PBS containing 10 % normal rabbit serum and 0.1 % Tween 20, and 10 $\mu$g/ml of peroxidase, diluted with the above PBS, is added to the microplate, and incubated at room temperature for 1 hour. After removing the reaction mixture and washing three times with the above PBS, it is colored by adding the substrate ABTS in the same manner as described in Example 1. The calibration curve is prepared by repeating the above procedure except that a solution (100 $\mu$l per well) of a standard HGH in a PBS containing 1 % BSA and 0.1 % Tween 20 is used instead of the test sample. The following Table 4 shows the value of calibration curve.

Table 4

| HGH (ng/ml) | 0.4 | 0.8 | 1.6 | 3.2 | 6.4 |
|---|---|---|---|---|---|
| Average of measured value (A 405)* | 0.019 | 0.246 | 0.443 | 0.669 | 0.994 |

*) Background value A405 = 0.744 (corrected)

Example 4

To a cleaned microplate made of polyvinyl chloride which has 96 wells, (the same kind as the one used in Example 1) a solution of a rabbit anti-interferon-$\alpha$ (IFN-$\alpha$) antiserum (100 $\mu$g/ml) (which is purified by affinity column packed with IFN-$\alpha$ as a ligend) in a phosphate buffered saline (PBS) is added in an amount of 100 $\mu$l per each well, and the solution is incubated at 37°C for 2 hours. After removing the reaction mixture, the microplate thus treated is dried at 37°C for 10 minutes, and

then, PBS (200 μl per well) containing 3 % bovine serum albumin (BSA) is added, and reacted at 37°C for 1 hour, by which the region unreacted with anti-IFN-α antiserum is blocked with BSA. After removing the buffer solution, the microplate is dried at 37°C for 10 minutes, washed three times with PBS, and thereto are added a test sample (100 μl per well) containing IFN-α and a mouse monoclonal anti-IFN-α antibody (a purified IgG, 3.3 μg/ml; 20 μl per well derived from clone WHC 46 manufactured by Wellcome Co., UK) in PBS containing 5 % BSA and 0.1 % Tween 20, and the mixture is incubated at 4°C overnight. After removing the reaction mixture, the microplate is washed three times with PBS, and 100 μl of a 50 fold-diluted rabbit anti-mouse immunoglobulin antibody, diluted with a PBS containing 10 % normal rabbit serum and 0.1 % Tween 20, is added to each well, and it is incubated at 37°C for 30 minutes. After washing three times with PBS, a mixture (100 μl per well) containing a 1,000 fold-diluted mouse anti-peroxidase antiserum, diluted with the above PBS, and 1 μg/ml of peroxidase, diluted with the above PBS, is added to the microplate, and incubated at 37°C for 30 minutes. After removing the reaction mixture, the microplate is washed three times with PBS, and thereto is added 100 μl per well of a substrate solution (12.5 mg/ml) of azino-bis(3-ethylbenzothiazoline-6-sulfonic acid) (ABTS) in a 100 mM sodium citrate-phosphate buffer (pH 5.0) solution containing 1.47 mM sodium perborate, and the mixture is colored by reacting at 37°C for 30 minutes. The absorbance of the colored product is measured at a wavelength of 405 nm, and the amount of IFN-$\alpha$ contained in the test sample is determined with a previously prepared calibration curve. The calibration curve is prepared by repeating the above procedure except that a solution of a standard IFN-$\alpha$ in a PBS containing 5 % BSA and 0.1 % Tween 20 is used instead of the test sample. The following Table 5 shows the value of calibration curve.

Table 5

| IFN-$\alpha$ (U/ml) | 2.8 | 28 | 280 | 2800 |
|---|---|---|---|---|
| Average of measured value (A 405)* | 0.039 | 0.220 | 0.810 | 1.541 |
| Error (± A 405) | 0.009 | 0.005 | 0.006 | 0.015 |

*)  Background value A405 = 0.300 (corrected)

As is shown in the above experimental data, the method of the present invention is effective for determination of IFN-$\alpha$ of 3U/ml or more with good reproduction, and the sensitivity for measurement is largely improved in comparison with the conventional method.

Example 5

In the same manner as described in Example 4, a test sample and a mouse monoclonal anti-IFN-$\alpha$ antibody are added to a microplate bound with a rabbit anti-IFN-$\alpha$ antiserum (purified by affinity column packed with IFN-$\alpha$) and BSA and, incubated at 4°C overnight. After washing three times with PBS, a mixture (100 $\mu$l per well) of a rabbit anti-mouse immunoglobulin antibody diluted in 100 folds with a PBS containing 10 % normal rabbit serum and 0.1 % Tween 20 and a peroxidase-labelled sheep anti-mouse immunoglobulin antibody diluted in 20 folds with the above PBS is added, and incubated at 37°C for 30 minutes. After removing the reaction mixture and further washing three times with PBS, a mixture (100 $\mu$l per well) containing a mouse anti-peroxidase antiserum diluted in 1,000 folds with a PBS containing 10 % normal rabbit serum and 0.1 % Tween 20 and 1 $\mu$g/ml of peroxidase, diluted with the above PBS is added to the microplate, and incubated at 37°C for 30 minutes. After removing the reaction mixture and washing three times with the above PBS, it is colored by adding the substrate ABTS in the same manner as described in Example 4. The calibration curve is prepared by repeating the above procedure except that a solution (100 $\mu$l per well) of a standard IFN in a PBS containing 5 % BSA and 0.1 % Tween 20 is used instead of the test sample. The following Table 6 shows the value of calibration curve.

Table 6

| IFN-$\alpha$ (U/ml) | 2.8 | 28 | 280 | 2800 |
|---|---|---|---|---|
| Average of measured value (A 405)* | 0.040 | 0.182 | 0.720 | 1.658 |
| Error (± A 405) | 0.012 | 0.021 | 0.049 | 0.010 |

*) Background value A405 = 0.165 (corrected)

Example 6

To a cleaned microplate made of polyvinyl chloride which has 96 wells (the same kind as the one used in Example 1) a solution of a rabbit anti-carcinoembryonic antigen (CEA) antiserum (purified IgG, manufactured by DAKO) (100

$\mu$g/ml) in a phosphate buffered saline (PBS) is added in an amount of 100 $\mu$l per each well, and the solution is incubated at 37°C for 2 hours. After removing the reaction mixture, the microplate thus treated is dried at 37°C for 10 minutes, and then, a PBS (200 $\mu$l per well) containing 3 % bovine serum albumin (BSA) is added, and reacted at 37°C for 1 hour, by which the region unreacted with anti-CEA antiserum is blocked with BSA. After removing the buffer solution, the microplate is dried at 37°C for 10 minutes, washed three times with PBS, and are added a test sample (100 $\mu$l per well) containing CEA and a mouse monoclonal anti-CEA antibody (purified IgG, 4 $\mu$g/ml, 20 $\mu$l per well) in a PBS containing 5 % BSA and 0.1 % Tween 20, and the mixture is incubated at 4°C overnight. After removing the reaction mixture, the microplate is washed three times with PBS, and thereto is added a rabbit anti-mouse immunoglobulin antibody (100 $\mu$l per well) diluted in 100 folds with a PBS containing 10 % normal rabbit serum and 0.1 % Tween 20, and incubated at 37°C for 30 minutes. After washing three times with PBS, a mixture (100 $\mu$l per well) containing a 1,000 fold diluted mouse anti-peroxidase antiserum, diluted with the above PBS, and 1 $\mu$g/ml of peroxidase, diluted with the above PBS, is added to the microplate, and incubated at 37°C for 30 minutes. After removing the reaction mixture, the microplate is washed three times with PBS, and thereto is added a substrate solution (12.5 mg/ml, 100 $\mu$l per well) of azino-bis(3-ethylbenzothiazoline-6-sulfonic acid) (ABTS) in a 100 mM sodium citrate-phosphate buffer (pH 5.0) solution containing 1.47 mM sodium perborate, and the mixture is colored by reacting at 37°C for 30 minutes. The absorbance of the colored product is measured at a wavelength of 405 nm, and the amount of CEA contained in the test sample is determined with a previously prepared calibration curve. The calibration curve is prepared by repeating the above procedure except that a solution of a standard CEA in a PBS containing 5 % BSA and 0.1 % Tween 20 is used instead of the test sample. The following Table 7 shows the value of calibration curve.

Table 7

| CEA (ng/ml) | 0.2 | 0.6 | 1.8 | 5.4 | 16.2 |
|---|---|---|---|---|---|
| Average of measured value (A 405)* | 0.239 | 0.500 | 0.779 | 1.094 | 1.410 |
| Error (± A 405) | 0.004 | 0.022 | 0.001 | 0.054 | 0.043 |

*) Background value A405 = 0.207 (corrected)

As is shown in the above experimental data, the method of the present invention is effective for determination of CEA of 0.2 ng/ml or more with good reproduction, and the sensitivity for measurement is improved in comparison with the conventional method.

Example 7

To a cleaned microplate made of polyvinyl chloride which has 96 wells, a solution of a rabbit anti-CEA antiserum (purified IgG , manufactured by DAKO) (100 $\mu$g/ml) in a phosphate buffered saline (PBS) is added in an amount of 100 $\mu$l per each well, and the solution is incubated at 37°C for 2 hours. After removing the reaction mixture, the microplate thus treated is dried at 37°C for 10 minutes, and then, a PBS (200 μl per well) containing 3 % bovine serum albumin (BSA) is added, and reacted at 37°C for 1 hour, by which the region unreacted with anti-CEA antiserum is blocked with BSA. After removing the buffer solution, the microplate is dried at 37°C for 10 minutes, washed three times with PBS, and thereto are added a test sample (100 μl per well) containing CEA and a mouse monoclonal anti-CEA antibody (manufactured by Hybritech; purified IgG, 4 μg/ml, 20 μl per well) in a PBS containing 5 % BSA and 0.1 % Tween 20, and the mixture is incubated at 4°C overnight. After removing the reaction mixture, the microplate is washed three times with PBS, and thereto is added a rabbit anti-mouse immunoglobulin antibody (100 μl per well) diluted in 100 folds with a PBS containing 10 % normal rabbit serum and 0.1 % Tween 20, and it is incubated at 37°C for 30 minutes. After washing three times with PBS, a mixture (100 μl per well) of a 1,000 fold-diluted mouse anti-peroxidase antiserum, diluted with the above PBS, and 1 μg/ml of peroxidase, diluted with the above PBS, is added to the microplate, and incubated at 37°C for 30 minutes. After removing the reaction mixture, the microplate is washed three times with PBS, and thereto is added a substrate solution (12.5 mg/ml, 100 μl per well) of azino-bis(3-ethylbenzothiazoline-6-sulfonic acid) (ABTS) in a 100 mM sodium citrate-phosphate buffer (pH 5.0) solution containing 1.47 mM sodium perborate, and the mixture is colored by reacting at 37°C for 30 minutes. The absorbance of the colored product is measured at a wavelength of 405 nm, and the amount of CEA contained in the test sample is determined with a previously prepared calibration curve. The calibration curve is prepared by repeating the above procedure except that a solution of a standard CEA in a PBS containing 5 % BSA and 0.1 % Tween 20 is used instead of the test sample. The following Table 8 shows the value of calibration curve.

Table 8

| CEA    (ng/ml) | 0.067 | 0.2 | 0.6 | 1.8 |
|---|---|---|---|---|
| Average of measured value (A 405)* | 0.323 | 0.593 | 0.829 | 1.025 |
| Error (± A 405) | 0.005 | 0.010 | 0.012 | 0.057 |

*)  Background value A405 = 0.218 (corrected)

As is shown in the above experimental data, the method of the present invention is effective for determination of CEA of 67 pg/ml or more with good reproduction, and the sensitivity for measurement is largely improved in comparison with the conventional method.

CLAIMS:

1. A method for the quantitative analysis of antigen, which comprises binding (1) an insoluble carrier or a coated insoluble carrier with (2) an antibody which is capable of recognizing an antigen (first antibody), (3) an antigen to be determined, (4) an antibody which is capable of recognizing an antigen originating from an animal species different from the animal species of the first antibody (second antibody), (5) an anti-immunoglobulin which is capable of recognizing an immunoglobulin of the same animal species as that of the second antibody, (6) an anti-enzyme antibody prepared by using the same animal species as that of the second antibody, and (7) an enzyme in this order, measuring the amount of the enzyme in the resulting enzyme-anti-enzyme antibody complex, and then determining the amount of the antigen therefrom.

2. A method as claimed in claim 1 wherein either one or both of the first antibody and the second antibody is a monoclonal antibody.

3. A method as claimed in claim 1, wherein the anti-immunoglobulin antibody which is capable of recognizing the immunoglobulin is a mixture of an anti-immunoglobulin antibody labelled with an enzyme and a nonlabelled anti-immunoglobulin antibody.

4. A method as claimed in any one of the preceding claims wherein the antigen to be determined is a growth hormone and factor, a tumor-associated antigen, or an interferon.